# EUROPÄISCHE PATENTSCHRIFT

(11) **EP 0 795 538 B1**
(45) Veröffentlichungstag und Bekanntmachung des Hinweises auf die Patenterteilung: **15.09.1999**
(21) Anmeldenummer: 97104180.1
(22) Anmeldetag: 12.03.1997
(51) Int. Cl.: C07C 67/60, C07C 69/716

(54) **Verfahren zur Herstellung von reinem Alkylacetessigsäurealkylestern**
Process for the preparation of pure alkyl esters of acetoacetic acid
Procédé de fabrication d'esters alkyliques purs d'acide acétoacétique

(30) Priorität: 13.03.1996 CH 65596; 30.04.1996 CH 108796
(43) Veröffentlichungstag der Anmeldung: 17.09.1997
(73) Patentinhaber: LONZA AG, CH-3945 Gampel/Wallis (CH)
(72) Erfinder: Jackson, Barry, Dr., 3902 Brig-Glis (Kanton Wallis) (CH)
(74) Vertreter: Winter, Brandl, Fürniss, Hübner, Röss, Kaiser, Polte, Kindermann Partnerschaft

(56) Entgegenhaltungen:
- EP-A- 0 100 019
- DE-A- 2 060 443

## Beschreibung

Die vorliegende Erfindung betrifft ein Verfahren zur Herstellung von reinen Alkylacetessigsäurealkylestern der allgemeinen Formel 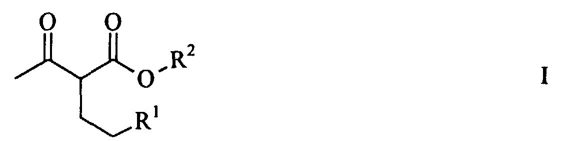 worin R¹ eine C₁-C₁₀-Alkylgruppe und R² eine C₁-C₄-Alkylgruppe bedeutet, aus Alkylacetessigsäurealkyllestern, welche als Verunreinigung Alkenylacetessigsäurealkylester der allgemeinen Formel 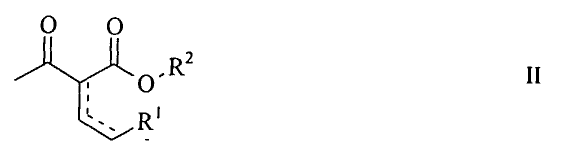 worin R¹ und R² die genannte Bedeutung haben, und gegebenenfalls weitere Nebenprodukte enthalten.

Alkylacetessigsäurealkylester sind wichtige Zwischenprodukte zur Herstellung von Pharmazeutika. Beispielsweise ist der Hexylacetessigsäuremethylester ein wichtiges Zwischenprodukt zur Herstellung von Tetrahydrolipstatin (Helv. Chim. Acta, Vol 70, (1987), 196-200).

Bekannt ist z. B. die Herstellung von Hexylacetessigsäuremethylester ausgehend von Hexanal und Acetessigsäuremethylester. Dabei wird Hexanal und Acetessigsäuremethylester z. B. durch klassische Knoevenagel-Reaktion (vgl. z. B. Organikum, 1976, S. 571) zum Hexenylacetessigsäuremethylester umgesetzt, welcher dann in Gegenwart von Wasserstoff und Hydrierkatalysatoren zum Endprodukt hydriert wird (DE-A 20 60 443). Ein solches Verfahren hat jedoch den Nachteil, dass der Hexylacetessigsäuremethylester mit schwierig abtrennbarem Hexenylacetessigsäuremethylester verunreinigt wird.

Aufgabe der vorliegenden Erfindung war daher, ein einfaches Reinigungsverfahren für Alkylacetessigsäurealkylester zur Verfügung zu stellen, wobei hohe Reinheit erreicht wird.

Diese Aufgabe wird durch das Reinigungsverfahren gemäss Anspruch 1 gelöst.

Erfindungsgemäss wird der Alkylacetessigsäurealkylester der allgemeinen Formel 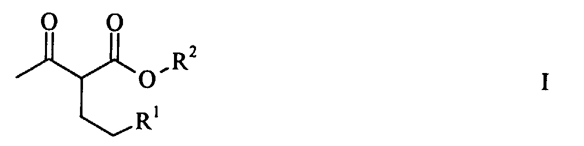 worin R¹ und R² die genannte Bedeutung haben, aus Alkylacetessigsäurealkylester, welcher mit einem Alkenylacetessigsäurealkylester der allgemeinen Formel 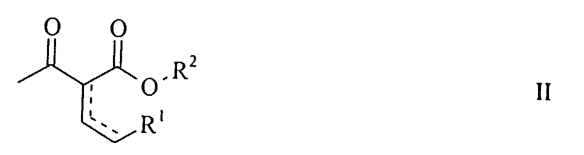 worin R¹ und R² die genannte Bedeutung haben und gegebenenfalls mit weiteren Nebenprodukten verunreinigt ist, derart gereinigt, dass man den Alkenylacetessigsäurealkylester der allgemeinen Formel II mit einem Ester der allgemeinen Formel 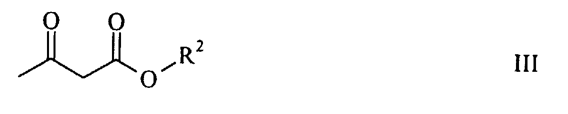 worin R² die genannte Bedeutung hat, in Gegenwart einer starken Base, in ein Zwischenprodukt überführt, welches abgetrennt wird.

Die Überführung des Alkenylacetessigsäurealkylesters in das Zwischenprodukt kann durch die an sich bekannte Michael-Reaktion (vgl. z. B. Organikum, 1976, S. 632) erfolgen. Als Zwischenprodukt wird zweckmässig eine Verbindung der allgemeinen Formel 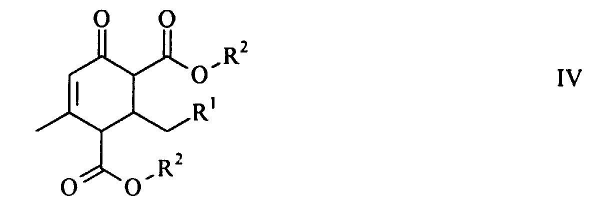 erhalten.

Der Rest R¹ bedeutet C₁-C₁₀-Alkyl wie z. B. Methyl, Ethyl, Propyl, i-Propyl, Butyl, t-Butyl, i-Butyl, Pentyl, Hexyl, Heptyl, Octyl, Nonyl oder Decyl. Vorzugsweise bedeutet R¹ Butyl oder Hexyl. Der Rest R² bedeutet C₁-C₄-Alkyl wie Methyl, Ethyl, Propyl, i-Propyl, Butyl, t-Butyl oder i-Butyl, vorzugsweise Methyl.

Die Ester der allgemeinen Formel III wie z. B. Acetessigsäuremethylester sind käufliche Verbindungen

Als starke Base können Alkalimetallalkoholate verwendet werden. Als Alkalimetallalkoholat kann Natrium- oder Kalium-methanolat, -ethanolat, -propanolat oder -butanolat, verwendet werden Zweckmässig wird das dem Ester entsprechende Alkalimetallalkoholat verwendet, vorzugsweise hat die starke Base einen pKₐ-Wert von grösser als 8.

Zweckmässig wird die starke Base in den gleichen Mol-Mengen wie die im Produkt vorhandene Verunreinigung (Alkenylacetessigsäurealkylester) eingesetzt.

Zweckmässig wird die Umsetzung des Alkenylacetessigsäurealkylesters (Formel II) mit dem Ester der allgemeinen Formel III bei einer Temperatur von 50 bis 150 °C, vorzugsweise bei einer Temperatur von 80 bis 110 °C durchgeführt.

Nach einer üblichen Umsetzungszeit von 1 bis 5 h wird die Verbindung der allgemeinen Formel IV oder deren decarboxyalkylierte Derivate erhalten.

Die Zwischenverbindungen der allgemeinen Formel IV werden dann zweckmässig durch Destillation abgetrennt.

Die Destillation wird vorzugsweise unter vermindertem Druck durchgeführt.

Durch diese Verfahrensweise werden reine Alkylacetessigsäurealkylester gewonnen, die mit weniger als 0,4% an Alkenylacetessigsäurealkylester verunreinigt sind.

### Beispiel 1

### Herstellung von 2-Hexylacetessigsäuremethylester

Das Reaktionsgemisch aus der Kondensation von Acetessigsäuremethylester (193,5 g) und Hexanal (151,8 g) mit Piperidin-Katalyse wurde in einem Autoklav bei 50 °C mit Wasserstoff und Pd/C-Katalyse hydriert. Nach Ende der Hydrierung und Abfiltrieren des Pd/C-Katalyts wurde das Zwei-Phassen Gemisch bei einem Druck von 15 mbar und einer Temperatur von 70 °C eingeengt Der Rückstand (262 g) enthielt noch 1,2% 2-Hexenylacetessigsäuremethylester.
Dann wurde Acetessigsäuremethylester (3,14 g) und Natriummethanolat (1,46 g einer 5,4 molaren Lösung) addiert und dieses Gemisch auf 90 °C während 5 Stunden erhitzt. Am Ende dieser Behandlung wurde noch 0,39% 2-Hexenylacetessigsäuremethylester gemessen. Das rohe Produkt wurde dann am Hochvakuum (3 - 4 mbar) in einem Dünnschichtverdampfer destilliert. Das 2-Hexylacetessigsäuremethylester-Destillat (240,9 g), mit einem Gehalt von 97,8%, enthielt noch 0,26% 2-Hexenylacetessigsäuremethylester.

### Beispiel 2

### Herstellung von 2-Butylacetessigsäuremethylester

Das Reaktionsgemisch aus der Kondensation von Acetessigsäuremethylester (1,65 Mol) und Butyraldehyd (1,5 Mol) mit Piperidin-Katalyse wurde in einem Autoklav bei 50 °C mit Wasserstoff und Pd/C-Katalyse hydriert. Nach Ende der Hydrierung und Abfiltrieren des Pd/C-Katalyts wurde das Zwei-Phasen Gemisch bei einem Druck von 15 mbar und einer Temperatur von 70 °C eingeengt. Der Rückstand (246,33 g) enthielt noch 2,16% 2-Butenylacetessigsäuremethylester und 5.0% Acetessigsäuremethylester.
Hierzu wurde Natriummethanolat (5,32 g einer 5,4 molaren Lösung) addiert und dieses Gemisch auf 90 °C während ca. 4 Stunden erhitzt. Am Ende dieser Behandlung wurde noch 0,16% 2-Butenylacetessigsäuremethylester gemessen. Das rohe Produkt wurde dann am Hochvakuum (3 - 4 mbar) in einem Dünnschichtverdampfer destilliert. Das 2-Butylacetessigsäuremethylester-Destillat (217,93 g), mit einem Gehalt von 94,4%, enthielt weniger als 0,1% 2-Butenylacetessigsäuremethylester.
Das Produkt konnte in einer Ausbeute von 72% bez. auf Butanol und mit einer Reinheit von 99,8% erhalten werden. Nach einem einfachen Fraktionierungsverfahren war der 2-Butenylacetessigsäuremethylester-Gehalt -Gehalt kleiner als 0,1%.

## Patentansprüche

1. Verfahren zur Herstellung von reinen Alkylacetessigsäurealkylestern der Formel 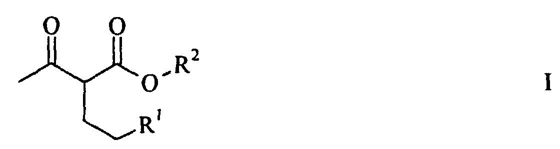 worin R¹ eine C₁-C₁₀-Alkylgruppe und R² eine C₁-C₄-Alkylgruppe bedeutet aus Alkylacetessigsäurealkylestern, welche Alkenylacetessigsäurealkylester der allgemeinen Formel 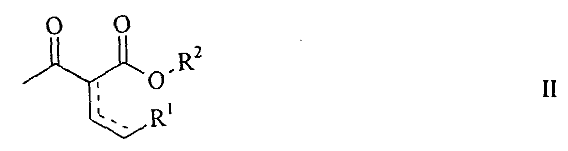 worin R¹ und R² die genannte Bedeutung haben, und gegebenenfalls weitere Nebenprodukte enthalten, dadurch gekennzeichnet, dass man den Alkenylacetessigsäurealkylester der Formel II mit einem Ester der allgemeinen Formel 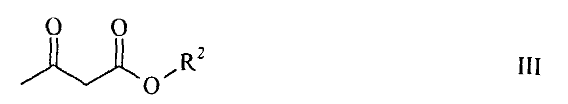 worin R² die genannte Bedeutung hat, in Gegenwart einer starken Base, in ein Zwischenprodukt überführt, welches abgetrennt wird.

2. Verfahren nach Patentanspruch 1, dadurch gekennzeichnet, dass man als starke Base ein Alkalimetallalkoholat verwendet.

3. Verfahren nach Patentanspruch 1 oder 2, dadurch gekennzeichnet, dass man als Ester der allgemeinen Formel III Acetessigsäuremethylester verwendet.

4. Verfahren nach mindestens einem der Patentansprüche 1 bis 3, dadurch gekennzeichnet, dass die Umsetzung des Alkenylacetessigsäurealkylesters (Formel II) mit dem Ester der allgemeinen Formel III bei einer Temperatur von 50 bis 150 °C durchgeführt wird.

## Claims

1. Process for the preparation of pure alkyl alkylacetoacetates corresponding to the formula 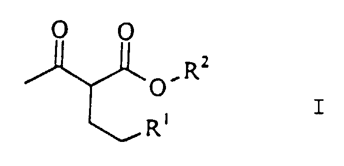 wherein R¹ denotes a C₁-C₁₀ alkyl group and R² denotes a C₁-C₄ alkyl group, from alkyl alkylacetoacetates which contain alkyl alkenylacetoacetates corresponding to the general formula 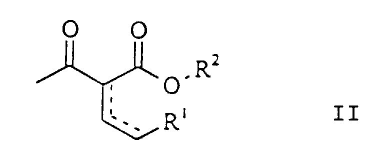 wherein R¹ and R² have the meaning given above, and possibly other secondary products, characterised in that the alkyl alkenylacetoacetate corresponding to formula II, together with an ester corresponding to the general formula 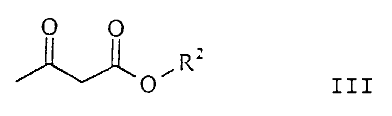 wherein R² has the meaning given above, in the presence of a strong base, is converted into an intermediate product, which is separated off.

2. Process according to claim 1, characterised in that an alkali metal alcoholate is used as the strong base.

3. Process according to claim 1 or 2, characterised in that methyl acetoacetate is used as the ester corresponding to the general formula III.

4. Process according to at least one of claims 1 to 3, characterised in that the reaction of the alkyl alkenylacetoacetate (formula II) with the ester corresponding to the general formula III is carried out at a temperature of 50°C to 150°C.

## Revendications

1. Procédé de préparation d'alkylacétoacétates d'alkyle purs de formule 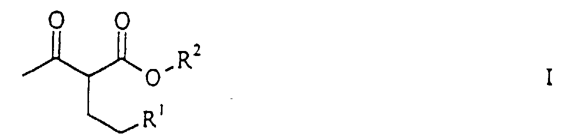 où R¹ représente un groupe alkyle en C₁-C₁₀ et R² représente un groupe alkyle en C₁-C₄ à partir d'alkylacétoacétates d'alkyle qui contiennent des alcénylacétoacétates d'alkyle de formule générale 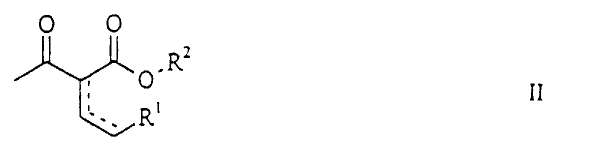 où R¹ et R² ont la signification citée, et éventuellement d'autres sous-produits, caractérisé en ce que l'on convertit l'alcénylacétoacétate d'alkyle de formule II avec un ester de formule générale 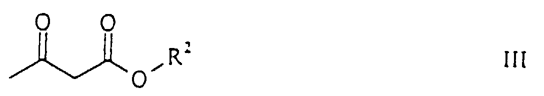 où R² a la signification citée, en présence d'une base forte, en un produit intermédiaire qui est séparé.

2. Procédé selon la revendication 1 caractérisé en ce que l'on utilise comme base forte un alcoolate de métal alcalin.

3. Procédé selon la revendication 1 ou 2 caractérisé en ce que l'on utilise comme ester de formule générale III l'acétoacétate de méthyle.

4. Procédé selon au moins l'une des revendications 1 à 3 caractérisé en ce que la réaction de l'alcénylacétoacétate d'alkyle (formule II) avec l'ester de formule générale III est conduite à une température de 50 à 150°C.
